# FASCICULE DE BREVET EUROPEEN

(11) **EP 0 641 559 B1**
(45) Date de publication et mention de la délivrance du brevet: **09.04.1997**
(21) Numéro de dépôt: 94401744.1
(22) Date de dépôt: 28.07.1994
(51) Int. Cl.: A61K 7/48, A61K 7/047

(54) **Produit pour enlever le vernis à ongles**
Nagellackentferner
Nail polish remover

(30) Priorité: 07.09.1993 FR 9310587
(43) Date de publication de la demande: 08.03.1995
(73) Titulaire: L'OREAL, 75008 Paris (FR)
(72) Inventeur: Junino, Alex, F-93190 Livry Gargan (FR); Ser, Jean-Claude, F-94150 Chevilly Larue (FR)
(74) Mandataire: Dodin, Catherine

(56) Documents cités:
- EP-A- 0 009 691
- US-A- 4 735 798
- 'The merck index. Eleventh edition' 1989 , MERCK & CO., INC , RAHWAY, N.J. U.S.A. Methyl Acetate. Number 5932 * page 948 *
- H. FEY & I. OTTE 'Wörterbuch der Kosmetik' 1985 , WISSENSCHAFTLICHE VERLAGSGESELLSCHAFT MBH , STUTTGART * page 178 *

## Description

La présente invention concerne de nouvelles compositions cosmétiques aqueuses, convenant notamment à l'élimination, par dissolution, des films de polymères tels que des films de vernis déposés à la surface des ongles, ainsi que leur utilisation.

Pour diverses raisons liées entre autres à la protection de l'environnement ou à la sécurité des consommateurs, on cherche actuellement de plus en plus, en particulier dans le domaine des compositions qui sont destinées à la solubilisation et à l'élimination des films de polymères déposés sur différents supports, comme par exemple les dissolvants pour vernis à ongles, à diminuer, voire supprimer, l'utilisation de certaines substances organiques jugées indésirables, en particulier de certains solvants.
C'est ainsi que, dans le cadre spécifique des compositions cosmétiques destinées à l'élimination des couches de vernis à ongles, un intérêt tout particulier s'est manifesté ces derniers temps pour essayer de réaliser des compositions dissolvantes de type aqueux, c'est à dire des compositions dans lesquelles les deux solvants organiques les plus couramment utilisés à ce jour, à savoir l'acétone et/ou l'acétate d'éthyle, sont, pour partie au moins, remplacés par de l'eau.
Or, à bien des égards, les compositions dissolvantes aqueuses qui ont été proposées jusqu'à présent ne donnent pas encore pleinement satisfaction. En effet, pour convenir à une application spécifiquement cosmétique du type de celle mentionnée ci-dessus, un dissolvant se doit de présenter certaines caractéristiques ou propriétés contraignantes, parmi lesquelles on peut plus particulièrement citer, et ceci de manière non limitative, d'abord une très bonne compatibilité/innocuité à l'égard des différentes matières constitutives de l'ongle et/ou de la peau, ensuite une bonne rapidité d'évaporation, et enfin un bon pouvoir dissolvant vis à vis des films nitrocellulosiques et autres déposés sur l'ongle.
D'une manière générale, il est souvent difficile, dans la pratique, de trouver, ou de mettre au point, un dissolvant susceptible de combiner tous ces avantages. Ainsi, des dissolvants à base d'acétone et d'eau sont déjà connus, mais les propriétés très délipidantes de l'acétone tendent à extraire de l'ongle les lipides qu'il contient et donc à le fragiliser. Pour cette raison, on cherche maintenant à éviter autant que possible l'utilisation de l'acétone dans les dissolvants pour vernis à ongles.
D'autres systèmes dissolvants, tels que ceux à base d'étheralcools et d'eau, sont également connus, mais ils font appel soit à des solvants ne présentant pas toutes les garanties nécessaires au regard de l'innocuité (c'est le cas par exemple du méthoxyéthanol), soit à des solvants dont l'évaporation est trop lente (c'est le cas par exemple du méthoxyisopropanol), ce qui entraîne, dans ce dernier cas, un temps d'attente important entre l'étape de démaquillage de l'ongle (suivie éventuellement d'un nettoyage au savon) et l'étape d'application d'une nouvelle couche de vernis; cet inconvénient est évidemment peu compatible avec les impératifs de la vie active que connaissent aujourd'hui la plupart des utilisatrices de vernis à ongles, qui souhaitent pouvoir utiliser ce genre de produits en tous lieux et à tout moment.
Parmi les solvants organiques légers compatibles avec les exigences d'évaporation rapide d'une part et de bon pouvoir solvant du film d'autre part, on connaît certes, comme indiqué précédemment, I'acétate d'éthyle, mais ce composé n'autorise malheureusement pas une addition directe d'eau (problème de miscibilité); il est alors nécessaire soit de réaliser des systèmes émulsionnés complexes qui requièrent la présence d'agents émulsifiants, ces agents ayant pour inconvénient, entre autres, de laisser sur l'ongle un film gras qui, s'il n'est pas préalablement éliminé avec un nettoyage au savon, est incompatible avec la tenue sur l'ongle d'un nouveau film de vernis, soit d'associer l'acétate d'éthyle avec un ou des tiers solvants assurant la miscibilité avec l'eau, tel que par exemple de l'alcool éthylique, mais ces derniers composés présentant un faible pouvoir dissolvant vis à vis des films de résines, en particulier de nitrocellulose, leur taux d'introduction est assez limité et, par voie de conséquence, celui de l'eau également.
Une autre solution connue pour essayer d'introduire de l'eau en proportion suffisante dans les dissolvants pour vernis à ongles consiste à réaliser des compositions se présentant sous forme bi-phasique (phase organique-phase aqueuse), mais de telles compositions présentent notamment pour inconvénient d'une part de nécessiter une agitation énergique au moment de leur emploi de manière à former, préalablement à toute application, une dispersion qui soit la plus homogène possible, et, d'autre part, de ne pas toujours conserver des propriétés constantes au cours du temps (problème de décantation notamment). On voit donc qu'il existe actuellement dans l'état de l'art, et plus particulièrement dans le domaine des dissolvants pour vernis à ongles, un fort besoin quant à pouvoir disposer de compositions cosmétiques aqueuses, dissolvantes, non délipidantes, homogènes, stables, exemptes de tiers solvant et/ou de tensioactifs, et à évaporation rapide.

Le document EP-A-0009691 décrit une composition d'élimination des films de vernis à ongle.

La présente invention vise justement à la satisfaction d'un tel besoin.

Ainsi, à la suite d'importantes recherches menées sur la question, il a maintenant été trouvé par la demanderesse qu'il est possible d'obtenir des compositions présentant l'ensemble des caractéristiques et propriétés ci-dessus, en associant de l'acétate de méthyle avec de l'eau, la proportion pondérale en eau dans le mélange pouvant alors avantageusement atteindre des valeurs élevées. Cette découverte est à la base de la présente invention.

La présente invention a donc pour objet une composition cosmétique aqueuse dissolvante, notamment utilisable pour l'élimination des films de polymères tels que des films de vernis à ongles, ladite composition étant caractérisée par le fait qu'elle comprend un mélange d'acétate de méthyle et d'eau dans un rapport pondéral allant de 90/10 à 60/40.
De préférence, ce rapport est compris entre 80/20 et 70/30.
Il peut également varier selon la température de la composition.

Les compositions selon l'invention peuvent bien entendu contenir également des adjuvants cosmétiquement actifs, tels que ceux habituellement rencontrés dans les dissolvants pour vernis à ongle et qui sont en particulier destinés à améliorer l'état et/ou l'aspect de l'ongle.
Parmi ces adjuvants, on peut notamment citer :
- les alcools polyhydriques tels que la glycérine, le propylène glycol, l'éthylèneglycol, le propanediol-1,2 et l'érythritol,
- les acides aminés tels que la proline, l'arginine, la lysine, l'histidine, l'hydroxyproline et leurs dérivés,
- les vitamines, par exemple le D-panthénol et la vitamine H,
- les oligo-éléments, par exemple le magnésium, le manganèse, le fer, le zinc et le cuivre, ainsi que leurs différents sels, tels que gluconates.
On peut également ajouter en faibles quantités, généralement de l'ordre de 0,1 à 0,2 %, des huiles telles que l'huile d'amande douce, la lanoline et ses dérivés. On peut aussi introduire un ou des adjuvants choisis notamment parmi les agents conservateurs, tels que par exemple le parahydroxybenzoate de méthyle ou de butyle, les colorants tels que ceux référencés FD&C Yellow n°5, FD&C Red n°4, FD&C Blue n°1, D&C Green n°5, FD&C Green n°3, D&C Red n°33, FD&C Red n°40, D&C Yellow n°10, D&C Violet n°2, les parfums et les filtres UV. Les taux utilisés pour ces divers adjuvants doivent bien entendu rester compatibles avec la nécessité pour la composition d'une part de conserver l'ensemble de ses propriétés avantageuses pour la dissolution et l'élimination des films de polymères, et, d'autre part, de ne pas laisser sur l'ongle un film dont la nature pourrait altérer l'application ou la tenue ultérieures d'un vernis à ongles.

La composition selon l'invention peut se présenter sous une forme liquide, mais aussi sous la forme de gels grâce à l'addition d'agents gélifiants classiques. Ainsi, à titre d'agents gélifiants, on peut notamment citer les argiles modifiées telles que la bentone, les polymères de cellulose tels que les hydroxyalkylcelluloses et la carboxyméthylcellulose, les alginates, les gommes, les homopolymères ou copolymères acryliques, les copolymères vinylpyrrolidone/vinylacétate. Par ailleurs, si l'on souhaite obtenir une composition opalescente, on peut y ajouter des additifs appropriés tels que par exemple des dérivés styrène/acrylate.

Comme indiqué précédemment, les compositions selon l'invention, compte tenu de leurs caractéristiques et/ou propriétés, sont particulièrement utiles comme, ou pour la fabrication de, dissolvants pour films de vernis à ongles.
A titre de vernis à ongles susceptibles d'être efficacement éliminés grâce aux compositions selon l'invention, on peut notamment citer ceux à base de nitrocellulose, et/ou de polymères acryliques, et/ou de résines alkydes, et/ou de copolymères chlorés greffés résultant du greffage d'au moins une polyoléfine chlorée avec un ou des monomères insaturés du type acrylique, styrénique et/ou vinylique.

Des exemples concrets, mais nullement limitatifs, illustrant l'invention vont maintenant être donnés.

### Exemple 1

On donne ici un exemple de composition dissolvante pour vernis à ongles conforme à l'invention :

| | |
|---|---|
| - Acétate de méthyle | 72 g |
| - Glycérine | 3 g |
| - D-panthénol | 0,5 g |
| - Colorants | qs |
| - Eau déminéralisée qsp | 100 g |

### Exemple 2

On donne ici un autre exemple de composition dissolvante pour vernis à ongles conforme à l'invention :

| | |
|---|---|
| - Acétate de méthyle | 80 g |
| - Opacifiant⁽¹⁾ | 0,1 g |
| - Huile d'avocat | 0,2 g |
| - Glycérine | 1 g |
| - Colorants | qs |
| - Eau déminéralisée qsp | 100 g |

| | |
|---|---|
| ⁽¹⁾: mélange copolymère styrène/acrylate/divinylbenzène et nonoxynol-4 sulfate d'ammonium | |

### Exemple 3

On donne ici un autre exemple de composition dissolvante pour vernis à ongles conforme à l'invention :

| | |
|---|---|
| - Acétate de méthyle | 70 g |
| - Gluconate de zinc | 0,0005 g |
| - Gluconate de magnésium | 0,0005 g |
| - Colorants | qs |
| - Eau déminéralisée qsp | 100 g |

### Exemple 4

On donne ici un autre exemple de composition dissolvante pour vernis à ongles conforme à l'invention :

| | |
|---|---|
| - Acétate de méthyle | 75 g |
| - Glycérine | 3 g |
| - Eau déminéralisée | 21,89 g |
| - Biotine | 0,01 g |
| - Opacifiant⁽¹⁾ | 0,1 g |

| | |
|---|---|
| ⁽¹⁾: mélange copolymère styrène/acrylate/divinylbenzène et nonoxynol-4 sulfate d'ammonium | |

Cette composition a permis d'obtenir, qualitativement, de très bon résultats (bonne dissolution/élimination du vernis, rapidité d'évaporation, absence de film gênant résiduel) au niveau du démaquillage d'ongles sur lesquels était revêtu un film de vernis à ongles classique à base de nitrocellulose.

## Revendications

1. Composition cosmétique aqueuse dissolvante, notamment de films de polymères, comprenant un mélange d'acétate de méthyle et d'eau dans lequel les proportions pondérales entre l'acétate de méthyle et l'eau sont comprises entre 90/10 et 60/40.

2. Composition cosmétique selon la revendication 1, dans laquelle lesdites proportions sont comprises entre 80/20 et 70/30.

3. Composition cosmétique selon l'une quelconque des revendications précédentes, se présentant sous une forme homogène et stable.

4. Composition cosmétique selon l'une quelconque des revendications précédentes, se présentant sous la forme d'un liquide ou d'un gel.

5. Composition cosmétique selon l'une quelconque des revendications précédentes, exempte d'agents tensioactifs.

6. Composition cosmétique selon l'une quelconque des revendications précédentes, exempte de tiers solvants organiques.

7. Composition cosmétique selon l'une quelconque des revendications précédentes, contenant des adjuvants cosmétiquement acceptables.

8. Composition cosmétique selon la revendication 7, dans laquelle lesdits adjuvants sont choisis, seuls ou en mélanges, parmi les agents traitants de l'ongle, les agents conservateurs, les colorants, les parfums, les filtres UV, les agents gélifiants, les agents opalescents, les huiles végétales.

9. Utilisation d'une composition telle que définie à l'une quelconque des revendications précédentes comme, ou pour la fabrication de, dissolvant de films de polymères.

10. Utilisation selon la revendication 9, dans laquelle lesdits films de polymères sont des films de vernis à ongle.

11. Utilisation selon la revendication 10, dans laquelle lesdits films de vernis à ongles sont à base de nitrocellulose, et/ou de polymères acryliques, et/ou de résines alkydes, et/ou de copolymères chlorés greffés résultant du greffage d'au moins une polyoléfine chlorée avec un ou des monomères insaturés du type acrylique, styrénique et/ou vinylique.

12. Procédé d'élimination de films de polymères déposés sur des supports, consistant à appliquer sur lesdits films une composition telle que définie à l'une quelconque des revendications 1 à 8.

13. Procédé selon la revendication 12, pour l'élimination des films de vernis déposés sur des ongles.

14. Procédé selon la revendication 13, dans lequel lesdits films de vernis à ongles sont à base de nitrocellulose, et/ou de polymères acryliques, et/ou de résines alkydes, et/ou de copolymères chlorés greffés résultant du greffage d'au moins une polyoléfine chlorée avec un ou des monomères insaturés du type acrylique, styrénique et/ou vinylique.

## Claims

1. Aqueous dissolving cosmetic composition, in particular which dissolves polymer films, comprising a mixture of methyl acetate and water in which the weight proportions between the methyl acetate and the water are between 90/10 and 60/40.

2. Cosmetic composition according to Claim 1, in which the said proportions are between 80/20 and 70/30.

3. Cosmetic composition according to either of the preceding claims, which is in a stable, homogeneous form.

4. Cosmetic composition according to any one of the preceding claims, which is in the form of a liquid or a gel.

5. Cosmetic composition according to any one of the preceding claims, which is free of surfactants.

6. Cosmetic composition according to any one of the preceding claims, which is free of organic intermediary solvents.

7. Cosmetic composition according to any one of the preceding claims, which contains cosmetically acceptable adjuvants.

8. Cosmetic composition according to Claim 7, in which the said adjuvants are chosen, alone or as mixtures, from nail-treating agents, preserving agents, dyes, fragrances, UV screening agents, gelling agents, opalescent agents and plant oils.

9. Use of a composition as defined in any one of the preceding claims as, or for the manufacture of, an agent for dissolving polymer films.

10. Use according to Claim 9, in which the said polymer films are films of nail varnish.

11. Use according to Claim 10, in which the said films of nail varnish are based on nitrocellulose and/or on acrylic polymers and/or on alkyd resins and/or on grafted chlorinated copolymers resulting from the grafting of at least one chlorinated polyolefin with one or more unsaturated monomers of the acrylic, styrene and/or vinyl type.

12. Process for removing polymer films deposited on supports, which consists in applying a composition as defined in any one of Claims 1 to 8 to the said films.

13. Process according to Claim 12, for removing films of varnish deposited on the nails.

14. Process according to Claim 13, in which the said films of nail varnish are based on nitrocellulose and/or on acrylic polymers and/or on alkyd resins and/or on grafted chlorinated copolymers resulting from the grafting of at least one chlorinated polyolefin with one or more unsaturated monomers of the acrylic, styrene and/or vinyl type.

## Patentansprüche

1. Wäßrige, insbesondere Polymerfilme lösende kosmetische Zusammensetzung, die ein Gemisch von Methylacetat und Wasser enthält, wobei das Gewichtsverhältnis von Methylacetat zu Wasser im Bereich von 90/10 bis 60/40 liegt.

2. Kosmetische Zusammensetzung nach Anspruch 1, bei der das Gewichtsverhältnis im Bereich von 80/20 bis 70/30 liegt.

3. Kosmetische Zusammensetzung nach einem der vorhergehenden Ansprüche, die in homogener und stabiler Form vorliegt.

4. Kosmetische Zusammensetzung nach einem der vorhergehenden Ansprüche, die in Form einer Flüssigkeit oder als Gel vorliegt.

5. Kosmetische Zusammensetzung nach einem der vorhergehenden Ansprüche, die keine grenzflächenaktiven Stoffe enthält.

6. Kosmetische Zusammensetzung nach einem der vorhergehenden Ansprüche, die kein weiteres organisches Lösungsmittel enthält.

7. Kosmetische Zusammensetzung nach einem der vorhergehenden Ansprüche, die kosmetisch akzeptable Hilfsstoffe enthält.

8. Kosmetische Zusammensetzung nach Anspruch 7, bei der die Hilfsstoffe, die alleine oder im Gemisch eingesetzt sind, unter den Mitteln zur Behandlung der Nägel, Konservierungsmitteln, Färbemitteln, Parfums, UV-Filtern, Gelbildnern, Trübungsmitteln und pflanzlichen Ölen ausgewählt sind.

9. Verwendung einer Zusammensetzung nach einem der vorhergehenden Ansprüche als Lösungsmittel für Polymerfilme oder zur Herstellung von Lösungsmitteln für Polymerfilme.

10. Verwendung nach Anspruch 9, wobei die Polymerfilme Nagellackfilme sind.

11. Verwendung nach Anspruch 10, wobei die Nagellackfilme Filme auf Nitrocellulosebasis und/oder Acrylpolymerbasis und/oder Alkydharzbasis und/oder auf der Basis von chlorierten gepfropften Copolymeren, die durch Pfropfung mindestens eines chlorierten Polyolefins mit einem oder mehreren ungesättigten Monomeren vom Acryltyp, Styroltyp und/oder Vinyltyp erhalten sind, darstellen.

12. Verfahren zum Entfernen von auf Trägern aufgebrachten Polymerfilmen, das darin besteht, auf die Filme eine Zusammensetzung nach einem der Ansprüche 1 bis 8 aufzutragen.

13. Verfahren nach Anspruch 12 zur Entfernung von auf Nägeln aufgebrachten Nagellackfilmen.

14. Verfahren nach Anspruch 13, wobei die Nagellackfilme Filme auf Nitrocellulosebasis und/oder Acrylpolymerbasis und/oder Alkydharzbasis und/oder auf der Basis von chlorierten gepfropften Copolymeren, die durch Pfropfung mindestens eines chlorierten Polyolefins mit einem oder mehreren ungesättigten Monomeren vom Acryl-, Styrol- und/oder Vinyltyp erhalten sind, darstellen.
